# EUROPEAN PATENT SPECIFICATION

(11) **EP 3 950 016 B1**
(45) Date of publication and mention of the grant of the patent: **24.06.2026**
(21) Application number: 21306097.3
(22) Date of filing: 06.08.2021
(51) Int. Cl.: A61L 2/18, B60N 3/02

(54) **DISINFECTION SYSTEM FOR A VEHICLE FOR PUBLIC TRANSPORT**
DESINFEKTIONSYSTEM FÜR EIN FAHRZEUG DES ÖFFENTLICHEN VERKEHRS
SYSTÈME DE DÉSINFECTION POUR VÉHICULE DE TRANSPORT PUBLIC

(30) Priority: 07.08.2020 IT 202000019732
(43) Date of publication of application: 09.02.2022
(73) Proprietor: IVECO FRANCE S.A.S., 69200 Vénissieux (FR)
(72) Inventor: CODRON, Stephane, 69008 LYON (FR)
(74) Representative: Studio Torta S.p.A.

(56) References cited:
- EP-A1- 3 378 498
- WO-A1-2020/152301
- CN-U- 205 652 010
- CN-U- 208 248 038
- JP-B1- 6 652 611
- KR-A- 20140 076 305

## Description

### TECHNICAL FIELD

The present invention relates to a disinfection system for support columns for public use, in particular a disinfection system for the columns of a vehicle for public transport, for example a bus.

### STATE OF THE PRIOR ART

Support columns are normally used in many vehicles, for example in road vehicles for public transport. These columns are necessary for passengers to hold on to avoid falling in the event of vehicle acceleration changes.

Nevertheless, given the large number of passengers who can travel in a vehicle throughout the day, the above-mentioned columns obviously get contaminated by passengers.

In fact, by leaning on or sneezing near the column, passengers can deposit germs on the latter (for example, viruses, bacteria) that could easily be transmitted to other passengers who will touch the same contaminated part of the column. This need is felt even more in the ongoing SARS-COV-2 pandemic.

To ensure an acceptable level of hygiene, these columns are cleaned with specific chemicals on a weekly basis defined by the operator.

The time required to clean all the columns of a vehicle at the end of the service is very long. Therefore, it is not possible to clean the columns several times during the day, as this would cause the vehicle to stop for too long, which would increase the stopping costs for public transport companies that have to provide the service.

In addition, the chemicals used to clean the columns are harmful to the personnel and the environment.

An example of a known disinfection system is illustrated in EP3378498 A1; however, this system may disturb passengers when in operation.

Other examples are disclosed in publications CN208248038 U, CN205652010 U, KR20140076305 A, WO2020152301 A1 or JP6652611 B1.

It is therefore still necessary to increase the possibility of cleaning quickly and economically, as well as in a way that respects people and the environment.

The object of the invention is to solve the above-mentioned drawbacks in a cost-effective and optimized way.

### SUMMARY OF THE INVENTION

The above-mentioned objects are achieved by means of a vehicle comprising a disinfection system for its support columns, as claimed in the attached independent claims.

Preferred embodiments of the invention are carried out according to the claims dependent on or related to the above-mentioned independent claims.

### BRIEF DESCRIPTION OF THE DRAWINGS

Further features and advantages of the invention will be apparent from the following description thereof, given by way of illustrative, non-limiting example, with reference to the attached drawings, wherein:
- Figure 1 is a perspective view showing the inside of a vehicle for public transport, comprising a column disinfection system according to the invention;
- Figure 2 is a schematic side view showing a column disinfection system according to the invention; and
- Figure 3 is a cross-section view along the cutting line III-III of the system in Figure 2.

### DETAILED DESCRIPTION

The present description relates to a road vehicle for public transport, in the case described herein, without any loss of generality thereby, a bus.

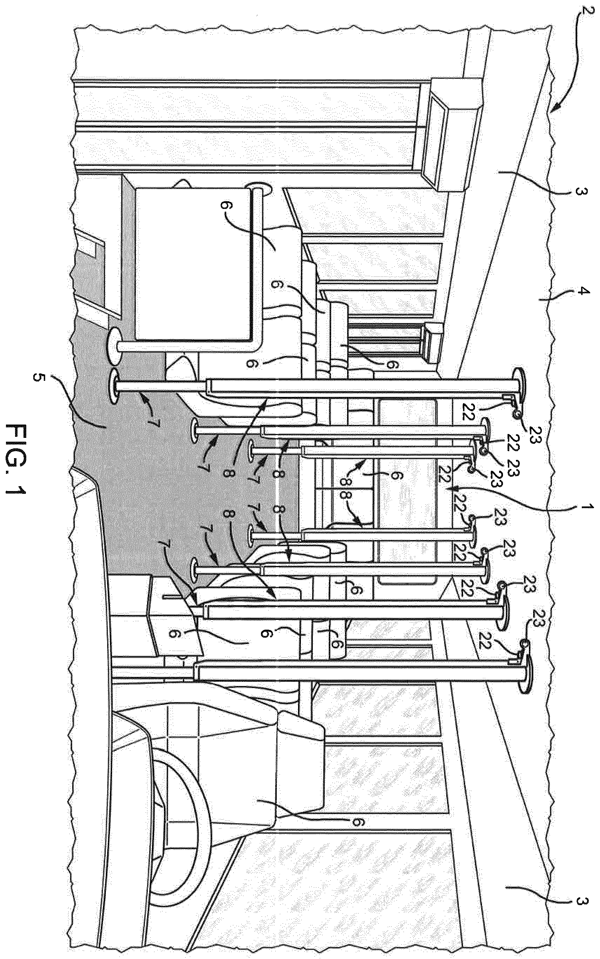

Figure 1 shows the internal space 1 of a bus 2 of a known type, delimited by a plurality of side walls 3, a roof 4, and a floor 5. The internal space 1 comprises a plurality of seats 6 suitable for allowing passengers to sit.

In a known manner and depending on the distribution of the seats 6, the bus 1 comprises a plurality of columns 7 configured to allow the passengers to grasp them during the trip in the bus 1.

Preferably, the columns 7 can be vertical, horizontal, or inclined columns and/or fittings. The columns 7 can be isolated columns such as those normally provided in front of the entrances of the bus 1 or columns 7 coupled together, such as those normally provided near the seats 2.

In a known manner, the columns 7 can consist of hollow tubes.

In the following description, reference will be made to a vertical column; however, it is clear that the present description can be applied to a horizontal or inclined column.

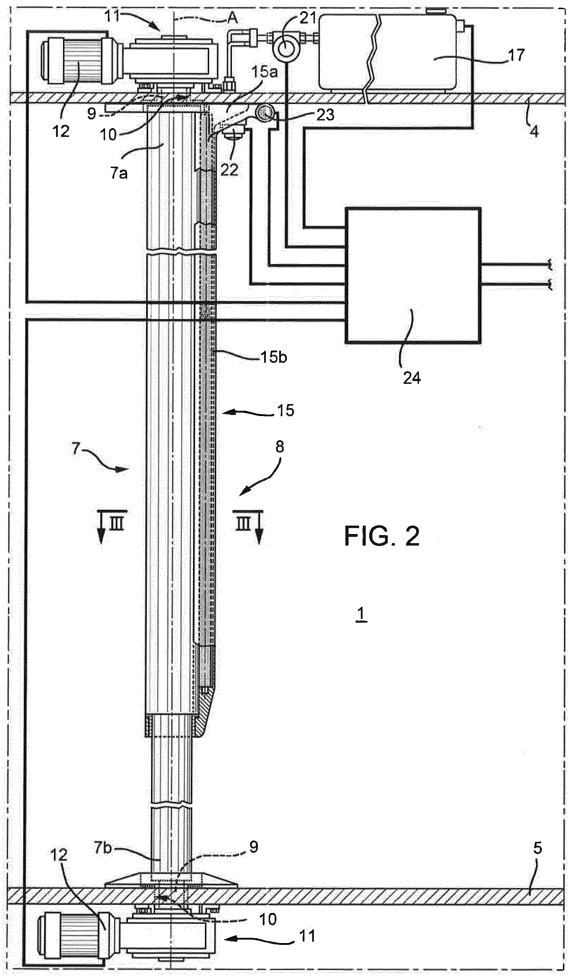

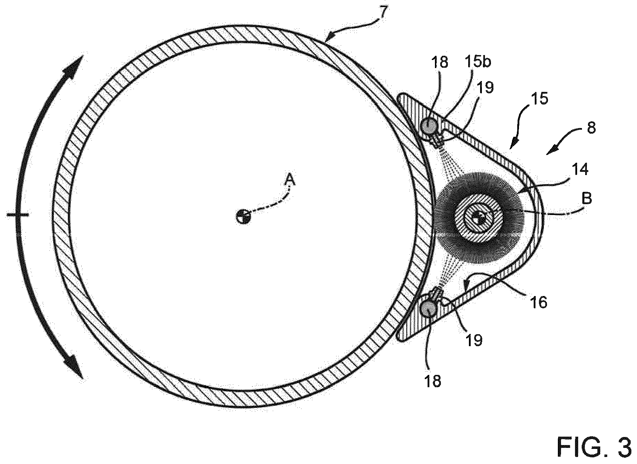

As shown in Figure 2, the column 7 extends longitudinally along an axis A between an upper portion 7a supported by the roof 4 and a lower portion 7b supported by the floor 5. Preferably, the column 7 has a curved, more preferably circular section (as shown in Figure 3).

According to the description the vehicle 1 comprises a disinfection system 8 configured to disinfect a predefined portion of a column 7, by dispensing disinfectant fluid onto the surface of this predefined portion. Advantageously, at least one of the column 7 and/or the disinfection system 8 is configured to rotate on itself to allow the disinfectant fluid to be distributed over the entire contact surface of the column 7.

In the case described herein, the column 7 is configured to rotate on itself, whereas the disinfection system 8 is fixed. Accordingly, the upper and lower portions 7a, 7b of the column 7 are supported so that they are free to rotate about the axis A with respect to the floor 5 and the roof 4, respectively.

In particular, the upper and lower portions 7a, 7b are fastened to the roof 4/the floor 5 by means of a coupling, such as for example a pin 9, as shown, configured to pivotally rotate in a respective opening 10 made in the floor 5 and in the roof 4, respectively.

The upper and lower portions 7a, 7b are connected to actuator means 11 configured to rotate the same, and therefore the whole column 7. In the case described herein, the upper portion 7a and the lower portion 7b are connected to actuator means 11.

Preferably, the actuator means 11 comprise an electric motor 12. In the case illustrated herein, each electric motor 12 is configured to rotate the respective pin 9. Preferably, the actuator means 11 are supported by the roof 4 and the floor 5, respectively, in a hidden manner with respect to the internal space 1.

The disinfection system 8 essentially comprises a brush 14, which is in contact with the surface of the column 7 and supported so that it is free to rotate about an axis B parallel to the axis A of the column 7.

Preferably, the brush 14 is supported by the roof 4 and housed in a fairing 15, which is fixed with respect to the roof 4 and configured to wrap the brush 14 along the axis B to prevent any contact with a passenger who could grasp the column 7.

The brush 14 can be supported so that it is free to rotate about the axis B and freely rotated by contact with the surface of the column 7 or coupled to the actuator means 11, which can make it rotate at the same speed as or at a different speed than the column 7, for example by virtue of a gear interposed between the column 7 and the actuator means 11.

Therefore, the fairing 15 comprises a fastening portion 15a configured to allow the attachment of the fairing 15 to the roof 4, and a covering portion 15b extending from the fastening portion 15a along the axis B towards the floor 5 so as to be in front of the entire outer surface of the column 7, which could be touched by a passenger.

The covering portion 15b defines a shape that is substantially tangential to the outer surface of the column 7, but without contact therewith, and defines a space 16 configured to house the above-mentioned brush 14.

The disinfection system 8 further comprises a source 17 of disinfectant fluid, for example an alcoholic solution, configured to be dispensed along the brush 14 to disinfect the surface of the column 7. The source 17 may be a tank, preferably supported by the roof 4 in a hidden manner with respect to the internal space 1 and preferably provided with means for detecting the level of the fluid.

The source 17 is fluidly connected to a plurality of openings 18 made along the fairing 15, advantageously in the covering portion 15b thereof, and configured to allow the disinfectant fluid to be sprayed onto the brush 14. Advantageously, each opening 18 is provided with a nozzle 19 configured to better distribute the disinfectant fluid on the brush 14.

In particular, the openings 18 are made in two rows on one side and the other of the brush 14 along the covering portion 15b.

The disinfection system 8 further comprises pumping means 21 configured to allow the passage of the disinfectant fluid from the source 17 towards the openings 18, preferably increasing its pressure.

The disinfection system 8 further comprises image acquisition means 22, for example at least one camera, configured to acquire an image of the entire column 7 and identify whether a passenger has touched its surface. Preferably, the image acquisition means 22 are supported by the fastening portion 15a and directed towards the floor 5 to acquire the entire contact surface of the column 7.

The disinfection system 8 further comprises signalling means 23 configured to signal the activation of the disinfection system 8. In the described embodiment, the signalling means 23 comprise a warning lamp configured to light up or change colour, thus indicating the availability of the column 7 or its unavailability due to the disinfection operation.

Preferably, the signalling means 23 are supported by the fairing 23, and preferably by the attachment system 15a, similarly to the image acquisition means 22.

The disinfection system also comprises an electronic unit 24 electronically connected to the actuator means 11, the source 17, the pumping means 21, the image acquisition means 22 and the signalling means 23. Preferably, this electronic unit is the ECU of the vehicle 1.

The electronic unit 24 comprises processing means configured to acquire data by means of the image acquisition means 22, and optionally the source 17, and accordingly control the actuator means 11 and the pumping means 21 to disinfect the column 7. If present, the electronic unit is also configured to control the signalling means 23 according to the activation of the actuator 11 and pumping means 21.

The electronic unit is therefore also configured to activate the pumping means 21 according to the data acquired by the image acquisition means 22 and according to the level of fluid in the source 17, which can be measured by the fluid level detection means.

The operation of a vehicle 1 comprising a disinfection system 8 as described herein above is as follows.

The image acquisition means 22 continuously detect the surface of the column 7. When they detect that a passenger has used a column 7 and has left, the electronic unit 24 activates the disinfection thereof.

Consequently, the actuator means 11 rotate the column 7 about the axis A, rotating the pin 9 and simultaneously the pumping means 21, thus sending disinfectant fluid to the brush 14. Disinfectant fluid is then sprayed onto the brush 14 through the nozzles 19 and this will be distributed on the surface of the column 7, with which it is in contact. In particular, the disinfectant fluid is deposited on the surface of the column 7 thanks to the centrifugal force due to the rotation of the brush 14 about the axis B. In fact, in the case described herein, the brush 14 is rotated, while remaining in contact with the surface of the column 7.

During the disinfection step, the signalling means 23 can switch from an off state to an on state by emitting a light indicating the operation of the disinfection system 8, or switch from a first colour to a second colour (e.g., green/red) for the same purpose.

After a predetermined time and/or a predetermined flow rate of disinfectant fluid sent to the brush 14, the electronic unit 24 controls the actuator means 11 to stop the rotation of the column 7 and/or of the brush 14.

Obviously, if someone grasps the column 7 when it is rotating, the torque resistant to the electric motors 12 creates an overcurrent that can be detected by the electronic unit 24, thus interrupting the disinfection step.

From the above, it is clear that the description also relates to a method for disinfecting a column 7 of a vehicle for public transport and comprising the following steps:
- identifying the need to disinfect a portion of a column 7;
- activating the disinfection system 8 in order to distribute disinfectant liquid on the column 7 and activating the rotation of the column 7 and/or of the disinfection system 8;
- controlling the disinfection system 8 in order to distribute a predetermined flow rate of disinfectant fluid or a flow rate of disinfectant fluid for a predetermined time; and
- stopping the rotation of the column 7 and/or of the disinfection system when the predetermined flow rate or timing has been reached.

The method can also comprise a further step of signalling the activation of the disinfection system 8.

The method may further comprise an automatic rotation inhibition step if it is detected that a passenger has grasped the column 7.

The advantages of a vehicle comprising a disinfection system according to the present invention are clear from the foregoing.

The disinfection system according to the invention allows the columns of a vehicle for public transport to be disinfected effectively, simply, and without disturbing the passengers.

In fact, the disinfection system can disinfect the entire column quickly, continuously, and automatically.

In fact, the use of a rotating column and a fixed fairing allows the column to be completely disinfected after each use of the column by a passenger without disturbing or harming him/her.

The use of a disinfectant fluid allows any type of pathogenic virus to be destroyed effectively.

The presence of the image acquisition means allows the automation of the disinfection system, and the presence of the signalling means allows the passenger to be warned of the disinfection operation.

Moreover, the hidden arrangement of the actuator means and the fluid source makes it possible to avoid reducing the available space inside the vehicle, except for the presence of the fairing.

Finally, it is clear that the vehicle comprising a disinfection system according to this invention may be subject to modifications, and variations may be created thereof, that, in any case, do not depart from the scope of protection defined by the claims.

The arrangement and number of the actuator means 11, the image acquisition means 22 and the signalling means 23 can vary according to the type of vehicle.

In addition, a source of disinfectant fluid and the pumping means could be fluidly connected to multiple fairings in order to supply the fluid to the disinfection systems of several columns of the vehicle.

## Claims

1. A vehicle (2) comprising at least one column (7) extending along a longitudinal axis (A) between a first and a second end portion (7a, 7b) thereof,
said first and a second end portion (7a, 7b) being respectively supported by between a roof (4) and a floor (5) of said vehicle (2), said vehicle (2) comprising a disinfection system (8) for said at least one column (7) and configured to dispense disinfectant fluid onto the outer surface of said column (7),
said disinfection system (8) comprising a brush (14) configured to be free to rotate about an axis (B) parallel to the axis (A) and in contact with said outer surface of said column (7), said brush (14) being supported by the roof (4) and housed in a fairing (15) configured to wrap the brush (14) along the axis (B), the fairing (15) comprising a fastening portion (15a) configured to allow the attachment of the fairing (15) to the roof (4) and a covering portion (15b) extending from the fastening portion (15a) along the axis (B) towards the floor (5) as to be in front of the entire surface of the column (7), which could be touched by a passenger, the covering portion (15b) defining a shape that is substantially tangential to the outer surface of the column (7), but without contact therewith, and defining a space (16) configured to house said brush (14) ,
wherein said column (7) is configured to rotate about said longitudinal axis (A) via actuator means (11)
to distribute said disinfectant fluid over the entire outer surface of said column (7) by means of said brush (14).

2. The vehicle according to claim 1, wherein said actuator means (11) are configured to rotate at least one of said first and second end portion (7a, 7b).

3. The vehicle according to claims 1 or 2, wherein said fairing (15) hides a plurality of openings (18) made in front of said brush (14), said disinfection system (8) comprising a source (17) of disinfectant fluid, said source (17) being fluidly connected to said openings (18).

4. The vehicle according to claim 3, wherein said disinfection system (8) comprises nozzles (19), each housed in a respective opening (18).

5. The vehicle according to one of claims 3 or 4, wherein said disinfection system (8) comprises pumping means (21) configured to send the disinfectant fluid from said source (17) to said openings (18).

6. The vehicle according to one of the preceding claims, wherein said disinfection system (8) comprises image acquisition means (22) configured to acquire an image of the column (7) and identify whether a passenger touches its surface, said disinfection system (8) being activated on the basis of said image.

7. The vehicle according to one of the preceding claims, wherein said disinfection system (8) comprises signalling means (23) configured to signal the activation of said disinfection system (8).

8. The vehicle according to claims 6 or 7, comprising an electronic unit (24) electronically connected to the actuator means (11) and to the image acquisition means (22), said electronic unit comprising processing means configured to acquire data by means of the image acquisition means (22) and consequently to control the actuator means (11) to allow the rotation of said column (7).

9. The vehicle according to claim 8, when dependent on claim 7, wherein said electronic unit (24) is electronically connected to said signalling means (23), said electronic unit (24) activating said signalling means (23) according to the activation of said disinfection system (8).

10. The vehicle according to claim 8, when dependent on claim 5, wherein said electronic unit (24) is electronically connected to said pumping means (21) and to said source (17), said electronic unit (24) activating said pumping means (21) according to the data acquired by said image acquisition means (22) and according to the level of fluid in said source (17).

11. A method of disinfecting a column of a vehicle for public transport of passengers according to one of claims 8 to 10, said method comprising the following steps, controlled by said electronic unit (24):
• identifying the need to disinfect said outer surface portion of a column (7);
• activating the disinfection system (8) to spread disinfectant liquid on said outer surface and activating the rotation of the column (7) ;
• controlling the disinfection system (8) in order to distribute a predetermined flow rate of disinfectant fluid and/or for a predetermined time; and
• stopping the rotation of the column (7) when said flow rate and/or said time has been reached.

12. The method according to claim 11, comprising a subsequent step of signalling the activation of the disinfection system (8).

13. The method according to one of claims 11 or 12, comprising a step of automatic inhibition of the rotation of said column (7) if it is detected that a passenger has gripped said outer surface portion of said column (7) .

## Patentansprüche

1. Fahrzeug (2), umfassend mindestens eine Säule (7), die sich entlang einer Längsachse (A) zwischen einem ersten und einem zweiten Endabschnitt (7a, 7b) davon erstreckt,
wobei der erste und ein zweiter Endabschnitt (7a, 7b) jeweils zwischen einem Dach (4) und einem Boden (5) des Fahrzeugs (2) getragen werden, wobei das Fahrzeug (2) ein Desinfektionssystem (8) für die mindestens eine Säule (7) umfasst und konfiguriert ist, um Desinfektionsfluid auf die Außenfläche der Säule (7) abzugeben,
wobei das Desinfektionssystem (8) eine Bürste (14) umfasst, die konfiguriert ist, um sich frei um eine Achse (B) parallel zur Achse (A) und in Kontakt mit der Außenfläche der Säule (7) zu drehen, wobei die Bürste (14) vom Dach (4) getragen wird und in einer Verkleidung (15) untergebracht ist, die konfiguriert ist, um die Bürste (14) entlang der Achse (B) zu wickeln, wobei die Verkleidung (15) einen Befestigungsabschnitt (15a) umfasst, der konfiguriert ist, um die Befestigung der Verkleidung (15) am Dach (4) zu ermöglichen, und einen Abdeckabschnitt (15b), der sich vom Befestigungsabschnitt (15a) entlang der Achse (B) in Richtung des Bodens (5) erstreckt, um sich vor der gesamten Oberfläche der Säule (7) zu befinden, die von einem Passagier berührt werden könnte, wobei der Abdeckabschnitt (15b) eine Form definiert, die im Wesentlichen tangential zur Außenfläche der Säule (7), aber ohne Kontakt damit ist, und einen Raum (16) definiert, der konfiguriert ist, um die Bürste (14) aufzunehmen,
wobei die Säule (7) konfiguriert ist, um sich über Betätigungsmittel (11) um die Längsachse (A) zu drehen, um das Desinfektionsfluid mittels der Bürste (14) über die gesamte Außenfläche der Säule (7) zu verteilen.

2. Fahrzeug nach Anspruch 1, wobei die Betätigungsmittel (11) konfiguriert sind, um mindestens einen des ersten und zweiten Endabschnitts (7a, 7b) zu drehen.

3. Fahrzeug nach Anspruch 1 oder 2, wobei die Verkleidung (15) eine Vielzahl von Öffnungen (18) verbirgt, die vor der Bürste (14) ausgebildet sind, wobei das Desinfektionssystem (8) eine Quelle (17) von Desinfektionsfluid umfasst, wobei die Quelle (17) fluidisch mit den Öffnungen (18) verbunden ist.

4. Fahrzeug nach Anspruch 3, wobei das Desinfektionssystem (8) Düsen (19) umfasst, die jeweils in einer jeweiligen Öffnung (18) untergebracht sind.

5. Fahrzeug nach einem der Ansprüche 3 oder 4, wobei das Desinfektionssystem (8) Pumpmittel (21) umfasst, die konfiguriert sind, um das Desinfektionsfluid von der Quelle (17) zu den Öffnungen (18) zu schicken.

6. Fahrzeug nach einem der vorhergehenden Ansprüche, wobei das Desinfektionssystem (8) Bilderfassungsmittel (22) umfasst, die konfiguriert sind, um ein Bild der Säule (7) zu erfassen und zu identifizieren, ob ein Passagier seine Oberfläche berührt, wobei das Desinfektionssystem (8) auf der Basis des Bildes aktiviert wird.

7. Fahrzeug nach einem der vorhergehenden Ansprüche, wobei das Desinfektionssystem (8) Signalisierungsmittel (23) umfasst, die konfiguriert sind, um die Aktivierung des Desinfektionssystems (8) zu signalisieren.

8. Fahrzeug nach Anspruch 6 oder 7, umfassend eine elektronische Einheit (24), die elektronisch mit den Betätigungsmitteln (11) und den Bilderfassungsmitteln (22) verbunden ist, wobei die elektronische Einheit Verarbeitungsmittel umfasst, die konfiguriert sind, um Daten mittels der Bilderfassungsmittel (22) zu erfassen und folglich die Betätigungsmittel (11) zu steuern, um die Drehung der Säule (7) zu ermöglichen.

9. Fahrzeug nach Anspruch 8, wenn abhängig von Anspruch 7, wobei die elektronische Einheit (24) elektronisch mit den Signalisierungsmitteln (23) verbunden ist, wobei die elektronische Einheit (24) die Signalisierungsmittel (23) gemäß der Aktivierung des Desinfektionssystems (8) aktiviert.

10. Fahrzeug nach Anspruch 8, wenn abhängig von Anspruch 5, wobei die elektronische Einheit (24) elektronisch mit den Pumpmitteln (21) und der Quelle (17) verbunden ist und die elektronische Einheit (24) die Pumpmittel (21) gemäß den von den Bilderfassungsmitteln (22) erfassten Daten und gemäß dem Fluidpegel in der Quelle (17) aktiviert.

11. Verfahren zum Desinfizieren einer Säule eines Fahrzeugs für den öffentlichen Personenverkehr nach einem der Ansprüche 8 bis 10, wobei das Verfahren die folgenden Schritte umfasst, die von der elektronischen Einheit (24) gesteuert werden:
• Feststellen der Notwendigkeit, den Außenflächenabschnitt einer Säule (7) zu desinfizieren;
• Aktivieren des Desinfektionssystems (8), um Desinfektionsflüssigkeit auf der Außenfläche zu verteilen, und Aktivieren der Drehung der Säule (7);
• Steuern des Desinfektionssystems (8), um eine vorbestimmte Durchflussmenge von Desinfektionsfluid und/oder für eine vorbestimmte Zeit zu verteilen; und
• Anhalten der Drehung der Säule (7), wenn die Durchflussmenge und/oder die Zeit erreicht wurde.

12. Verfahren nach Anspruch 11, umfassend einen nachfolgenden Schritt des Signalisierens 15 der Aktivierung des Desinfektionssystems (8).

13. Verfahren nach einem der Ansprüche 11 oder 12, umfassend einen Schritt des automatischen Hemmens der Drehung der Säule (7), wenn erfasst wird, dass ein Passagier den Außenflächenabschnitt der Säule (7) ergriffen hat.

## Revendications

1. Véhicule (2) comprenant au moins une colonne (7) s'étendant selon un axe longitudinal (A) entre des première et seconde parties d'extrémité (7a, 7b) de celui-ci,
lesdites première et seconde parties d'extrémité (7a, 7b) étant respectivement supportées entre un toit (4) et un plancher (5) dudit véhicule (2), ledit véhicule (2) comprenant un système de désinfection (8) pour ladite au moins une colonne (7) et configuré pour distribuer un fluide désinfectant sur la surface externe de ladite colonne (7),
ledit système de désinfection (8) comprenant une brosse (14) configurée pour être libre de tourner autour d'un axe (B) parallèle à l'axe (A) et en contact avec ladite surface externe de ladite colonne (7), ladite brosse (14) étant supportée par le toit (4) et logée dans un carénage (15) configuré pour envelopper la brosse (14) le long de l'axe (B), le carénage (15) comprenant une partie de fixation (15a) configurée pour permettre la fixation du carénage (15) au toit (4) et une partie de recouvrement (15b) s'étendant de la partie de fixation (15a) le long de l'axe (B) vers le plancher (5) pour être devant toute la surface de la colonne (7), pouvant être touchée par un passager, la partie de recouvrement (15b) définissant une forme sensiblement tangentielle à la surface externe de la colonne (7), mais sans contact avec celle-ci, et définissant un espace (16) configuré pour loger ladite brosse (14),
dans lequel ladite colonne (7) est configurée pour tourner autour dudit axe longitudinal (A) via des moyens d'actionneur (11)
pour répartir ledit fluide désinfectant sur toute la surface externe de ladite colonne (7) au moyen de ladite brosse (14).

2. Véhicule selon la revendication 1, dans lequel lesdits moyens d'actionneur (11) sont configurés pour faire tourner au moins l'une desdites première et seconde parties d'extrémité (7a, 7b).

3. Véhicule selon la revendication 1 ou 2, dans lequel ledit carénage (15) cache une pluralité d'ouvertures (18) réalisées devant ladite brosse (14), ledit système de désinfection (8) comprenant une source (17) de fluide désinfectant, ladite source (17) étant reliée fluidiquement auxdites ouvertures (18).

4. Véhicule selon la revendication 3, dans lequel ledit système de désinfection (8) comprend des buses (19), chacune logée dans une ouverture (18) respective.

5. Véhicule selon l'une des revendications 3 et 4, dans lequel ledit système de désinfection (8) comprend des moyens de pompage (21) configurés pour envoyer le fluide désinfectant depuis ladite source (17) vers lesdites ouvertures (18).

6. Véhicule selon l'une des revendications précédentes, dans lequel ledit système de désinfection (8) comprend des moyens d'acquisition d'image (22) configurés pour acquérir une image de la colonne (7) et identifier si un passager touche sa surface, ledit système de désinfection (8) étant activé sur la base de ladite image.

7. Véhicule selon l'une des revendications précédentes, dans lequel ledit système de désinfection (8) comprend des moyens de signalisation (23) configurés pour signaler l'activation dudit système de désinfection (8).

8. Véhicule selon la revendication 6 ou 7, comprenant une unité électronique (24) reliée électroniquement aux moyens d'actionneur (11) et aux moyens d'acquisition d'image (22), ladite unité électronique comprenant des moyens de traitement configurés pour acquérir des données par le biais des moyens d'acquisition d'image (22) et par conséquent pour commander les moyens d'actionneur (11) pour permettre la rotation de ladite colonne (7).

9. Véhicule selon la revendication 8, lorsqu'elle dépend de la revendication 7, dans lequel ladite unité électronique (24) est reliée électroniquement auxdits moyens de signalisation (23), ladite unité électronique (24) activant lesdits moyens de signalisation (23) selon l'activation dudit système de désinfection (8).

10. Véhicule selon la revendication 8, lorsqu'elle dépend de la revendication 5, dans lequel ladite unité électronique (24) est reliée électroniquement auxdits moyens de pompage (21) et à ladite source (17), ladite unité électronique (24) activant lesdits moyens de pompage (21) selon les données acquises par lesdits moyens d'acquisition d'image (22) et selon le niveau de fluide dans ladite source (17).

11. Méthode de désinfection d'une colonne d'un véhicule de transport public de passagers selon l'une des revendications 8 à 10, ladite méthode comprenant les étapes suivantes, commandées par ladite unité électronique (24) :
• l'identification de la nécessité de désinfecter ladite partie de surface externe d'une colonne (7) ;
• l'activation du système de désinfection (8) pour répandre du liquide désinfectant sur ladite surface externe et l'activation de la rotation de la colonne (7) ;
• la commande du système de désinfection (8) afin de distribuer un débit prédéterminé de fluide désinfectant et/ou pendant une durée prédéterminée ; et
• l'arrêt de la rotation de la colonne (7) lorsque ledit débit et/ou ledit temps est/sont atteint(s).

12. Méthode selon la revendication 11, comprenant une étape ultérieure de signalisation de l'activation du système de désinfection (8).

13. Méthode selon l'une des revendications 11 et 12, comprenant une étape d'interdiction automatique de la rotation de ladite colonne (7) s'il est détecté qu'un passager a saisi ladite partie de surface externe de ladite colonne (7).
